# EUROPEAN PATENT SPECIFICATION

(11) **EP 1 376 118 B1**
(45) Date of publication and mention of the grant of the patent: **16.06.2010**
(21) Application number: 03019159.7
(22) Date of filing: 18.10.1996
(51) Int. Cl.: G01N 27/419

(54) **A method of measuring the concentration of NO in a measurement gas**
Verfahren zur Messung der Konzentration von NO in einem Messgas
Procédé de mesure de la concentration de NO d'un gaz à mesurer

(30) Priority: 20.10.1995 JP 27245895
(43) Date of publication of application: 02.01.2004
(62) Divisional of application: 96307586.6
(73) Proprietor: NGK Insulators, Ltd., Nagoya-City, Aichi Pref. 467-8530 (JP)
(72) Inventor: Kato, Nobuhide, Ama-gun, Aichi Pref., 497 (JP)
(74) Representative: Paget, Hugh Charles Edward

(56) References cited:
- EP-A- 0 517 366
- EP-A- 0 678 740
- EP-A- 0 731 351
- EP-A- 0 769 693
- EP-A- 0 769 694
- US-A- 5 217 588
- US-A- 5 304 294

## Description

### BACKGROUND OF THE INVENTION

### Field of the Invention:

The present invention relates to a method of measuring NO. In particular, the present invention relates to improvement in a method directed to a combustion gas as a measurement gas, for measuring NO as a measurement gas component in such a gas.

### Description of the Related Art:

Various measuring methods and devices have been proposed for determining the concentration of NOx in a measurement gas. A known method, for example, employs a sensor comprising a Pt electrode and an Rh electrode formed on an oxygen ion-conductive solid electrolyte such as zirconia. This method utilizes the ability of Rh to reduce NOx so that an electromotive force generated between the two electrodes is measured. However, a problem arises in that such a sensor tends to suffer from an influence of noise, since the electromotive force varies to a great extent depending on a change in concentration of oxygen contained in a combustion gas as a measurement gas, while the electromotive force varies to a small extent in response to a change in concentration of NOx. On the other hand, a reducing gas such as CO is indispensable for such a sensor in order to induce the ability to reduce NOx. However, in general, a large amount of NOx is produced under a combustion condition concerning an excessively small amount of fuel, in which an amount of produced CO is less than the amount of produced NOx, resulting in a drawback that measurement cannot be performed for a combustion gas discharged under such a combustion condition.

Another method of measuring NOx is also known, based on a combination of a set of electrochemical pumping cell and sensing cell including Pt electrodes and oxygen-ion conductive solid electrolyte, and another set of electrochemical pumping cell and sensing cell including Rh electrodes and oxygen-ion conductive solid electrolyte, as disclosed in Japanese Laid-open Patent Publication Nos. 63-38154 and 64-39545. In this method, NOx is measured on the basis of a difference between values of pumping currents. EP-A-0257842 (corresponding to JP-A-63-38154) discloses porous coverings on the sensing electrodes as an alternative to the use of openings as diffusion resistances. Other methods are disclosed, for example, in Japanese Laid-open Patent Publication Nos. 1-277751 and 2-1543. In one method, two pairs, i.e., a first pair and a second pair of electrochemical pumping cell and sensing cell are prepared. A limiting pumping current is measured by using a sensor comprising the first pair of pumping and sensing cells, under a partial pressure of oxygen at which NOx is not reduced, while a limiting pumping current is measured by using a sensor comprising the second pair of pumping and sensing cells, under a partial pressure of oxygen at which NOx is reduced, so that a difference between the measured limiting pumping currents is determined. In another method, a sensor comprising a pair of pumping cell and sensing cell is used, in which a difference in limiting current is measured by switching the partial pressure of oxygen in a measurement gas between a partial pressure of oxygen at which NOx is reduced and a partial pressure of oxygen at which NOx is not reduced.

In the aforementioned methods of measuring NOx, however, an extremely small part of the value of the limiting current is based on the objective NOx, and the most part of the value of the limiting current is occupied by electric power caused by oxygen contained in a large amount in ordinary cases. Therefore, a small current value corresponding to NOx is determined from a difference between two large current values. Accordingly, in the case of the method based on the use of the one set of sensor, problems arise in that the NOx cannot be continuously measured, the operating response is inferior, and the accuracy is inferior. On the other hand, in the case of the method based on the use of the two sets of sensors, an error is likely to occur in a measured value if the oxygen concentration in a measurement gas greatly changes. Therefore, this method cannot be employed in automobile applications, for example, where the oxygen concentration in a measurement gas varies to a large extend. This inconvenience arises from the fact that the dependency of pumping current on oxygen concentration concerning one sensor is different from that concerning the other sensor. In the case of an automobile, for example, the oxygen concentration in exhaust gas is generally several percentages under a running condition of an air/fuel ratio of 20, whereas the NOx concentration is several hundreds of ppm. The concentration of NOx is about 1/100 of the concentration of oxygen. Therefore, only a slight difference in the dependency of pumping current on oxygen concentration brings about a situation in which a difference in the limiting current value corresponding to a change in oxygen concentration is larger than an amount of change in the limiting current based on NOx to be measured. In addition, if a diffusion rate-determining means formed in the pumping cell is clogged with oil ash in the exhaust gas, the pumping current may be undesirably changed, resulting in reduced accuracy. Further, if the temperature of the exhaust gas greatly varies, a measured value may involve some abnormality. Moreover, a difference in chronological change in any characteristic between the two sensors, if any, may directly lead to measuring errors, resulting in a drawback that the entire system is made undurable for use over a long period of time.

The oxygen present in the measurement gas causes various problems upon measurement of NOx, as described above. Accordingly, it has been strongly desired to solve these problems.

In order to solve the problems described above, the present inventors have revealed a new measuring system in Japanese Patent Application No. 7-48551 (corresponding to EP-A-0678740, published 25 October 1995 and to be considered under EPC Article 54(3)). In this system, a measurement gas component having bonded oxygen, such as NOx, contained in a measurement gas can be measured continuously and accurately with good response over a long period of time without being affected by the oxygen concentration in the measurement gas or any change thereof, by utilizing first and second electrochemical pumping cells arranged in series.

Namely, in the previously proposed new system, a measurement gas containing a measurement gas component having bonded oxygen to be measured is successively introduced from an external measurement gas-existing space into first and second processing zones under predetermined diffusion resistances respectively. At first, in the first processing zone, the partial pressure of oxygen is controlled to have a low value which does not substantially affect measurement of an amount of the measurement gas component by pumping out oxygen in the atmosphere by using a first electrochemical pumping cell. In the second processing zone, the measurement gas component in the atmosphere introduced from the first processing zone is reduced or decomposed. Oxygen produced by the reduction or decomposition is pumped out by the aid of an oxygen-pumping action effected by a second electrochemical pumping cell. Thus a pumping current flowing through the second electrochemical pumping cell is detected to obtain a detected value from which the amount of the measurement gas component in the measurement gas is determined.

However, as a result of further investigation by the present inventors on such a new measuring system, the sensor concerning the new measuring system described above has been clarified to have the following problem. Namely, measurement of a measurement gas component at a low concentration, for example, measurement of NOx at several ppm gives a pumping current of about several tens of nA detected by the second electrochemical pumping cell, which is small as a detection signal.

US-A-5217588 discloses an NOx sensor having two chambers linked by diffusion resistance means, in which an electrochemical sensor detects the voltage between two electrodes located in respective ones of said chambers and in which both electrodes are exposed to the same test gas.

### SUMMARY OF THE INVENTION

Thus the present invention has been made in order to solve the problems involved in the NOx sensor concerning the previously proposed new system for measuring the NOx concentration in the measurement gas. The invention can provide a method of measuring NO, in which a large change in signal can be obtained upon measurement of a concentration of low concentration NOx in a measurement gas, making it possible to perform measurement continuously and accurately with good response over a long period of time.

According to the present invention, there is provided a method of measuring an amount of NO as a gas component in a measurement gas, as set out in claim 1.

Features and advantages of the present invention will become more apparent from the following description when taken in conjunction with the accompanying drawings. A preferred embodiment of the present invention is described by way of illustrative example.

### DESCRIPTION OF THE DRAWINGS

FIG. 1 (A) explanatorily shows a plan view of a first NOx sensor described below, and FIG. 1 (B) explanatorily shows an enlarged view of principal components, taken along a cross section of A-A shown in FIG. 1 (A).
FIG. 2 (A) explanatorily shows a plan view corresponding to FIG. 1 (A) of a modified form of the NOx sensor, and FIG. 2 (B) explanatorily shows a cross-sectional view corresponding to FIG. 1 (B).
FIG. 3 (A) explanatorily shows a plan view corresponding to FIG. 1 (A) of another modified form of the NOx sensor, and FIG. 3 (B) explanatorily shows an enlarged view of principal components, taken along a cross section of B-B shown in FIG. 3 (A).
FIG. 4 (A) explanatorily shows a cross-sectional view corresponding to FIG. 1 (B) of still another modified form of the NOx sensor, and FIG. 4 (B) shows a block diagram illustrating a system of pumping voltage control effected in the NOx sensor shown in FIG. 4 (A).
FIG. 5 (A) explanatorily shows a plan view corresponding to FIG. 1 (A) of a second NOx sensor described below, and FIG. 5 (B) explanatorily shows a cross-sectional view corresponding to FIG. 1 (B).
FIG. 6 explanatorily shows a modified form of the second NOx sensor, explanatorily illustrating a cross-sectional view corresponding to FIG. 1 (B).
FIG. 7 shows a graph exemplarily illustrating relationships between the NO concentration in a measurement gas and the electromotive force, obtained in the first and second NOx sensors.

### DESCRIPTION OF THE PREFERRED EMBODIMENTS

In order to clarify the present invention more specifically, the system of the present invention will be explained in detail below with reference to the drawings.

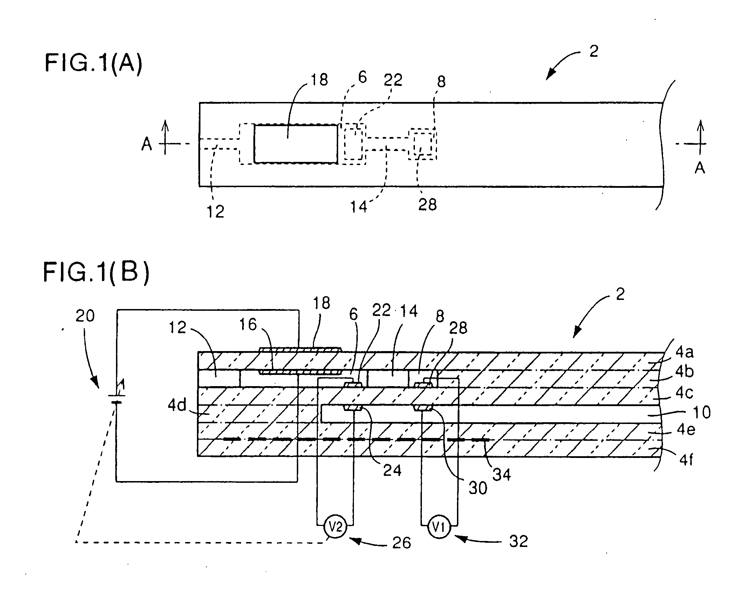

At first, FIG. 1 (A) and FIG. 1 (B) illustrate a representative example of a NOx sensor (first NOx sensor) used in a method of the present invention. FIG. 1 (A) shows a plan view of the sensor, and FIG. 1 (B) explanatorily shows an enlarged view of principal components, taken along a cross section of A-A shown in FIG. 1 (A).

With reference to FIG. 1 (A) and FIG. 1 (B), reference numeral 2 indicates a sensor element having a slender and lengthy plate-shaped configuration. As shown in FIG. 1 (B), the sensor element 2 is a plate-shaped object having an integrated structure comprising a plurality of dense and airtight oxygen ion-conductive solid electrolyte layers 4a, 4b, 4c, 4d, 4e, 4f stacked up with each other. Each of the solid electrolyte layers 4a to 4f is formed of a known oxygen ion-conductive solid electrolyte material such as zirconia ceramics. The sensor element 2 having the integrated structure is produced by firing stacked unfired solid electrolyte layers into the integrated structure in the same manner as those hitherto performed.

The sensor element 2 having the integrated structure described above includes a first internal space 6 and a second internal space 8 each having a rectangular and plane configuration and individually constructed such that the first internal space 6 is located on a distal side of the element. A reference air-introducing passage 10, which serves as a reference gas-existing space, extends in a longitudinal direction of the sensor element 2. The reference air-introducing passage 10 is provided independently from the first and second internal spaces 6, 8, while overlapping vertically with the first and second internal spaces 6, 8. The reference air-introducing passage 10 is open at the proximal end of the sensor element 2 to make communication with atmospheric air. In this embodiment, the first and second internal spaces 6, 8 are formed such that spaces corresponding thereto formed through the solid electrolyte layer 4b are closed by the upper and lower solid electrolyte layers 4a, 4c. Accordingly, the first and second internal spaces 6, 8 are substantially located on an identical plane. The reference air-introducing passage 10 is formed such that a space corresponding thereto formed through the solid electrolyte layer 4d is closed by the upper and lower solid electrolyte layers 4c, 4e.

A first diffusion rate-determining passage 12, which serves as a first diffusion rate-determining means for allowing the first internal space 6 to communicate with an external measurement gas-existing space, is formed by cutting out the solid electrolyte layer 4b so that the first diffusion rate-determining passage 12 is open at the distal end of the sensor element 2. A measurement gas containing NOx is introduced into the first internal space 6 under a predetermined diffusion resistance through the first diffusion rate-determining passage 12. A second diffusion rate-determining passage 14, which serves as a second diffusion rate-determining means for making communication between the two internal spaces 6,8, is formed through the solid electrolyte layer 4b located between the first and second internal spaces 6, 8. An atmosphere in the first internal space 6 is introduced into the second internal space 8 under a predetermined diffusion resistance through the second diffusion rate-determining passage 14.

An inner pumping electrode 16, which is composed of a rectangular porous cermet electrode, is provided on a portion of the solid electrolyte layer 4a exposed to the first internal space 6, in contact therewith. An outer pumping electrode 18, which is also composed of a rectangular porous cermet electrode, is provided on an outer surface portion of the solid electrolyte layer 4a corresponding to the inner pumping electrode 16, in contact therewith. An electrochemical pumping cell is constructed by the electrodes 16, 18 and the solid electrolyte layer 4a. A desired voltage is applied between the two electrodes 16, 18 of the electrochemical pumping cell by using an external variable power source 20, and a current is allowed to flow in a direction from the outer pumping electrode 18 to the inner pumping electrode 16. Thus oxygen in the atmosphere in the first internal space 6 can be pumped out to the external measurement gas-existing space. The porous cermet electrode comprises a metal such as Pt and a ceramic such as ZrO₂. However, the inner pumping electrode 16 is arranged in the first internal space 6, which contacts with the measurement gas. Therefore, it is necessary for the inner pumping electrode 16 to use a metal having a weak ability or no ability to reduce the NOx component in the measurement gas. Desirably, the inner pumping electrode 16 comprises, for example, a cermet of ZrO₂ and a Pt-Au alloy. As described above, the pumping electrodes 16, 18 generally have a porous structure.

A second measuring electrode 22, which is composed of a porous cermet electrode similarly to the inner pumping electrode 16, is provided on a portion of the solid electrolyte layer 4c exposed to the first internal space 6, in contact therewith. A second reference electrode 24, which is composed of a porous cermet electrode similarly to the outer pumping electrode 18, is provided on a portion of the solid electrolyte layer 4c exposed to the reference air-introducing passage 10, in contact therewith. An electrochemical cell, which serves as a partial oxygen pressure-detecting means, i.e., a second electrochemical sensor cell, is constructed by the second measuring electrode 22, the second reference electrode 24, and the solid electrolyte layer 4c. The partial pressure of oxygen in the atmosphere in the first internal space 6 is detected by measuring an electromotive force generated between the second measuring electrode 22 and the second reference electrode 24, by using a second potentiometer 26, on the basis of a difference in oxygen concentration between the atmosphere in the first internal space 6 and the reference air (atmospheric air) in the reference air-introducing passage 10, as well known in the art. The voltage of the variable power source 20 is controlled on the basis of a value of partial pressure of oxygen in the atmosphere in the first internal space 6, detected by the second potentiometer 26. Thus the pumping action of the electrochemical pumping cell is controlled so that the partial pressure of oxygen in the atmosphere in the first internal space 6 comes to a predetermined low value at which NOx is not substantially reduced in the presence of the inner pumping electrode 16 and the second measuring electrode 22 of the second electrochemical sensor cell.

A rectangular first measuring electrode 28 is provided in the second internal space 8, on a portion of the solid electrolyte layer 4c exposed to the second internal space 8, in contact therewith. The first measuring electrode 28 is composed of a porous cermet comprising zirconia as a ceramic and Rh which is a metal capable of reducing NOx. Thus the first measuring electrode 28 functions as a NOx-reducing catalyst capable of reducing NOx existing in an atmosphere in the second internal space 8. On the other hand, a first reference electrode 30 is provided on the solid electrolyte layer 4c, corresponding to the first measuring electrode 28, so that the first reference electrode 30 is exposed to the inside of the reference air-introducing passage 10. A first electrochemical sensor cell is constructed by the first measuring electrode 28, the first reference electrode 30, and the solid electrolyte layer 4c. As well known, an electromotive force, which is generated between the first measuring electrode 28 and the first reference electrode 30, is outputted on the basis of a difference in oxygen concentration between an atmosphere around the first measuring electrode 28 and an atmosphere around the first reference electrode 30. The electromotive force is measured by a first potentiometer 32. Thus it is possible to detect the partial pressure of oxygen in the atmosphere around the first measuring electrode 28, in other words, the partial pressure of oxygen defined by oxygen produced by reducing or decomposing the measurement gas component (NOx).

A heater 34, which is heated by external power supply, is embedded in the sensor element 2 such that the heater 34 is vertically interposed between the solid electrolyte layers 4e, 4f. Upper and lower surface of the heater 34 are covered with thin layers of ceramic such as alumina, although not shown, in order to obtain electric insulation from the solid electrolyte layers 4e, 4f. As shown in FIG. 1 (B), the heater 34 is arranged over the entire length ranging from the first internal space 6 to the second internal space 8. Thus the internal spaces 6, 8 are heated to a predetermined temperature respectively. Consequently, the electrochemical pumping cell, as a matter of course, as well as the first and second electrochemical sensor cells is heated to and maintained at the predetermined temperature respectively.

In conformity with the arrangement of the sensor element 2 as described above, its distal end is arranged in the measurement gas-existing space. Accordingly, the measurement gas is introduced into the first internal space 6 under the predetermined diffusion resistance through the first diffusion rate-determining passage 12 provided in the sensor element 2. The measurement gas introduced into the first internal space 6 undergoes the oxygen-pumping action evoked by applying a predetermined voltage between the two pumping electrodes 16, 18 which constitute the electrochemical pumping cell so that the partial pressure of oxygen is controlled to have a predetermined value, for example, 10⁻¹⁰ atm.

In order to control the partial pressure of oxygen in the atmosphere in the first internal space 6 to have the predetermined value, a technique is adopted on the basis of the well known Nernst equation. Namely, the electromotive force between the second measuring electrode 22 and the second reference electrode 24 of the electrochemical sensor cell is measured by using the second potentiometer 26. The voltage (variable power source 20) applied between the two electrodes 16, 18 of the electrochemical pumping cell is controlled so that the electromotive force is, for example, 430 mV (700 °C). Thus the partial pressure of oxygen is controlled to have the objective value of 10⁻¹⁰ atm. Namely, the voltage of the first electrochemical pumping cell is controlled so that the electromotive force corresponds to a difference between a desired oxygen concentration in the first internal space 6 and an oxygen concentration in the reference air. The first diffusion rate-determining passage 12 serves to reduce the amount of oxygen in the measurement gas diffusing and flowing into the measuring space (first internal space 6) when the voltage is applied to the first electrochemical pumping cell so that the current flowing through the electrochemical pumping cell is suppressed.

A state of partial pressure of oxygen, in which NOx in the atmosphere is not reduced by the inner pumping electrode 16 and the second measuring electrode 22, for example, a state of partial pressure of oxygen, in which the reaction: NO → 1/2N₂ + 1/2O₂ does not take place, is established in the first internal space 6 even under a heated condition caused by heating by the external measurement gas and the heater 34. If NOx in the measurement gas (or in the atmosphere) is reduced in the first internal space 6, it is impossible to accurately measure NOx in the second internal space 8. In this context, it is necessary to establish the state in the first internal space 6 in which NOx is not reduced by any component (any metal component of the inner pumping electrode 16 and the second measuring electrode 22 in this embodiment) which may concern reduction of NOx.

The measurement gas, which has its partial pressure of oxygen controlled in the first internal space 6 as described above, is introduced into the second internal space 8 through the second diffusion rate-determining passage 14 under the predetermined diffusion resistance. NOx in the measurement gas introduced into the second internal space 8 is reduced or decomposed in accordance with, for example, the reaction: NO → 1/2N₂ + 1/2O₂ around the first measuring electrode 28 which also functions as a catalyst for reducing NOx, under the heated condition and under the partial pressure of oxygen. The first potentiometer 32 measures an electromotive force generated between the first measuring electrode 28 and the first reference electrode 30 on the basis of a difference between the partial pressure of oxygen in the atmosphere around the first reference electrode 30 and a partial pressure of oxygen in the atmosphere around the first measuring electrode 28, i.e., in the atmosphere in the second internal space 8, defined by oxygen produced by the reduction or decomposition. Accordingly, it is possible to detect the partial pressure of oxygen in the atmosphere in the second internal space 8, in other words, the raised partial pressure of oxygen, defined by oxygen produced by the reduction or decomposition of NOx. Thus the NOx concentration in the measurement gas can be measured. In order to increase the ratio of change in partial pressure of oxygen in the atmosphere in the second internal space 8 based on oxygen produced as described above, it is desirable that the partial pressure of oxygen in the atmosphere in the first internal space 6, and consequently the partial pressure of oxygen in the atmosphere in the second internal space 8 in the absence of NOx is maintained at a sufficiently low value which is, for example, 10⁻¹⁰ atm.

The partial pressure of oxygen in the atmosphere in the second internal space 8, which is defined and raised by oxygen produced by reducing or decomposing NOx in the measurement gas, pretends to decrease on account of diffusion of oxygen in a direction to counteract the pressure gradient of partial pressure of oxygen between the second internal space 8 and the first internal space 6, i.e., from the second internal space 8 to the first internal space 6. However, in the NOx sensor the pressure gradient is not completely counteracted because of the predetermined diffusion resistance possessed by the second diffusion rate-determining passage 14. The partial pressure of oxygen in the atmosphere in the second internal space 8 is equilibrated at a value which is higher than the partial pressure of oxygen in the first internal space 6, substantially corresponding to the increased partial pressure of oxygen brought about by oxygen produced by reducing or decomposing NOx in the atmosphere in the second internal space 8, i.e., substantially corresponding to the NOx concentration in the measurement gas. Thus the electromotive force, which corresponds to the partial pressure of oxygen in the atmosphere in the second internal space 8, is detected from the output from the electrochemical sensor cell. Accordingly, even when the amount of produced oxygen is minute upon measurement of the NOx concentration at a low concentration, the NOx concentration can be measured as a large change in electromotive force.

The sensitivity for detecting the NOx concentration can be regulated by changing the cross-sectional area, the cross-sectional shape, and the length of the second diffusion rate-determining passage 14.

The sensitivity for detecting the NOx concentration can be further increased by packing a porous material having a predetermined diffusion resistance into the second diffusion rate-determining passage 14 and/or the second internal passage 8, because of the reason as described above.

In the foregoing, any one of the inner pumping electrode 16 and the second measuring electrode 22, arranged in the first internal space 6, is required not to reduce or decompose NOx in the atmosphere respectively at the ambient temperature and the controlled partial pressure of oxygen in the internal space respectively. Accordingly, those usable include electrode metals such as Au and Ni having no ability or a weak ability to reduce or decompose NOx. Those advantageously usable include, for example, cermet electrodes comprising the metal described above, and cermet electrodes obtained by using an alloy prepared by adding a metal having no catalytic ability such as Au and Ni described above to a noble metal such as Pt, Pd, and Rh. Desirably, the first measuring electrode 28 arranged in the second internal space 8 is a cermet electrode composed of, for example, Rh capable of reducing or decomposing NOx in the atmosphere at the environmental temperature and the partial pressure of oxygen in the second internal space 8. It is of course possible to use, as the first measuring electrode 28, those obtained by arranging and stacking, on an ordinary electrode, an Rh or Pt electrode, or a catalyst material comprising a NOx-reducing metal carried on a ceramic porous material such as alumina, and those obtained by arranging an Rh catalyst electrode on a Pt electrode.

In any case, the respective electrodes provided for the NOx sensor described above, especially the inner pumping electrode 16 and the measuring electrodes 22, 28 arranged in the respective internal spaces are desirably cermet electrodes composed of an electrode metal and an appropriate ceramic. In particular, as exemplified above, in the case of the use of the first measuring electrode 28 which also functions as a NOx-reducing catalyst, it is desirable to use a porous cermet electrode comprising a ceramic and a known metal capable of reducing NOx such as Rh and Pt. The NOx-reducing catalyst may be provided in the close vicinity of the first measuring electrode 28 of the first electrochemical pumping cell for detecting the partial pressure of oxygen in the second internal space 8. Alternatively, a porous alumina, on which a NOx-reducing catalyst comprising, for example, Rh is carried, may be stacked on the first measuring electrode 28 by means of printing or the like to form a NOx-reducing catalyst layer on the electrode.

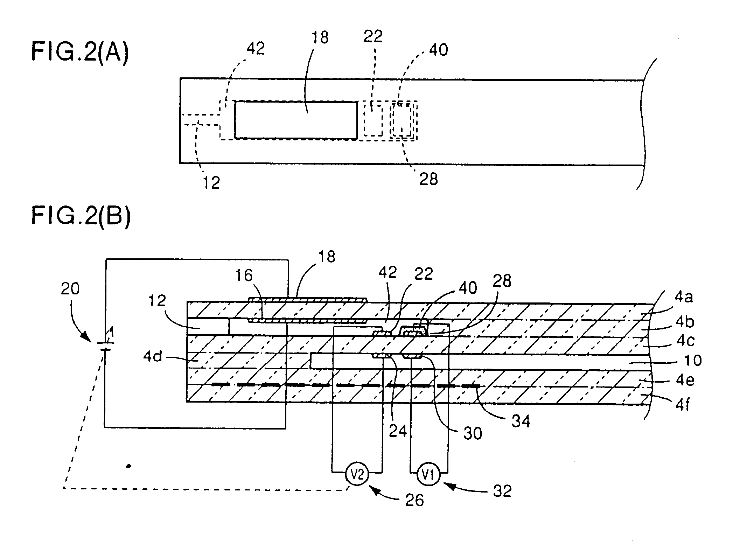

It is needless to say that the NOx sensor used should not be interpreted such that the NOx sensor is limited to only the structure of the foregoing description. One modified form of the NOx sensor is shown in FIG. 2 (A), (B).

The first modified form shown in FIG. 2 (A), (B) is different from the foregoing first sensor, which is specifically **characterized in that** the second internal space and the second diffusion rate-determining passage as the second diffusion rate-determining means are constructed by one porous material layer 40 having a predetermined diffusion resistance, arranged to cover a first measuring electrode 28, and the first measuring electrode 28 and a second measuring electrode 22 are arranged in series adjacent to one another with respect to a direction of diffusion of an atmosphere in an internal space 42. Namely, the porous material layer 40 is constructed to serve as both of the second diffusion rate-determining passage and the second internal space. The measurement gas, which has its partial pressure of oxygen controlled in the internal space 42, diffuses through the porous material layer 40 under .a predetermined diffusion resistance, and arrives at the first measuring electrode 28 arranged at a section under the porous material layer 40. Thus NOx in the measurement gas is reduced by the first measuring electrode 28. An electromotive force, which is defined by oxygen produced by the reduction of NOx, corresponding to a raised partial pressure of oxygen, is generated between the first measuring electrode 28 and the first reference electrode 30. The electromotive force is measured by a first potentiometer 32. Accordingly, the structure of the NOx sensor is simplified. Such a NOx sensor is advantageous from the viewpoint of cost and applicability to mass production.

It is desirable that the first measuring electrode 28 and the second measuring electrode 22 are arranged as nearly as possible in the diffusing direction. Accordingly, it is possible to decrease the change in electromotive force of the first electrochemical sensor cell, i.e., the measuring error for the NOx concentration, caused by the change in distribution of oxygen concentration existing in the gas-diffusing direction depending on the oxygen concentration in the measurement gas.

For example, porous Al₂O₃ and ZrO₂ are used for the porous material layer 40 having the predetermined diffusion resistance, arranged to cover the first measuring electrode 28.

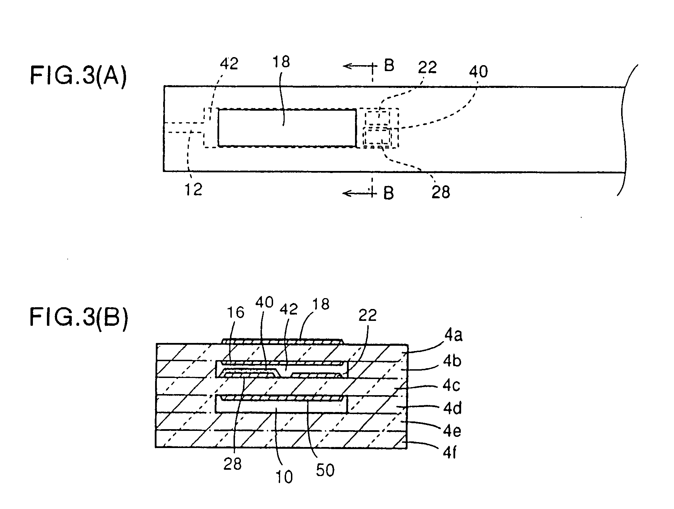

A second modified form shown in FIG. 3 (A), (B) is different from the first modified form shown in FIG. 2 (A) and FIG. 2 (B), which is specifically **characterized in that** a first measuring electrode 28 is covered with one porous material layer 40 having a predetermined diffusion resistance for constructing the second diffusion rate-determining means and the second internal space, the first measuring electrode 28 and a second measuring electrode 22 are arranged in parallel with respect to an atmosphere-diffusing direction in an internal space 42, and the first and second reference electrodes are provided as one common reference electrode 50. Owing to the arrangement of the first measuring electrode 28 and the second measuring electrode 22, it is possible to further decrease the change in electromotive force of the first electrochemical sensor cell, i.e., the measuring error for the NOx concentration, caused by the change in distribution of oxygen concentration existing in the gas-diffusing direction depending on the oxygen concentration in the measurement gas. In the second modified form, the first measuring electrode 28 and the second measuring electrode 22 may be provided on the solid electrolyte layers 4a, 4c respectively to oppose to one another with the internal space 42 interposed therebetween, while they are arranged in parallel with respect to the atmosphere-diffusing direction in the internal space 42.

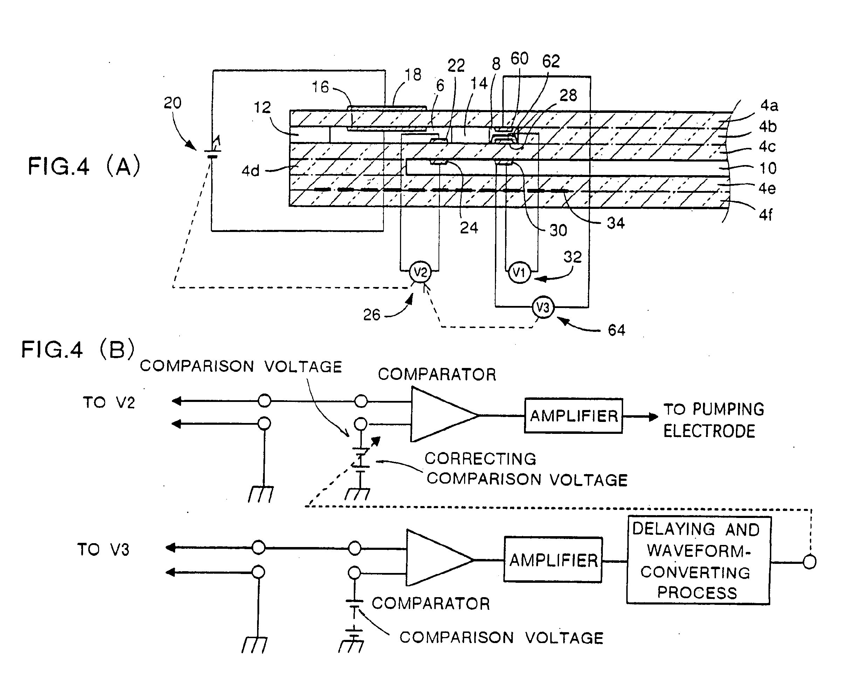

A third modified form shown in FIG. 4 (A) is specifically characterized as follows, in addition to the feature of the first embodiment shown in FIG. 1 (A), (B). Namely, a porous material layer 62 having a predetermined diffusion resistance is provided around the first measuring electrode 28 of the first electrochemical sensor cell, arranged in the second internal space 8. A third measuring electrode 60 is provided in contact with the solid electrolyte layer 4a located on the second internal space 8 so that the third measuring electrode 60 is opposed to the first measuring electrode 28 with the second internal space 8 interposed therebetween. The third measuring electrode 60, the solid electrolyte layers 4a, 4b, 4c, and the first reference electrode 30 constitute a third electrochemical sensor cell for detecting, in the second internal space 8, the partial pressure of oxygen in the atmosphere introduced from the first internal space 6 into the second internal space 8 through the second diffusion rate-determining passage 14. An electromotive force outputted from the third electrochemical sensor cell is detected by a third potentiometer 64 to obtain a detected value on the basis of which correction is performed for control of the electrochemical pumping cell effected by the second electrochemical sensor cell.

Namely, the partial pressure of oxygen in the measurement gas, which is introduced from the first internal space 6 into the second internal space 8 through the second diffusion rate-determining passage 14, is detected by the third potentiometer 64 on the basis of the electromotive force of the third electrochemical sensor cell constructed by the third measuring electrode 60 arranged in the second internal space 8, the first reference electrode 30, and the solid electrolyte layers 4a, 4b, 4c. The control of partial pressure of oxygen in the first internal space 6, which is performed by the electrochemical pumping cell controlled by the second electrochemical sensor cell, is corrected on the basis of a detected value of partial pressure of oxygen. The measurement gas introduced into the second internal space 8 diffuses under the predetermined diffusion resistance of the porous material layer 62 arranged in the second internal space 8, and arrives at the first measuring electrode 28 arranged at the inner section of the porous material layer 62. Thus NOx is reduced by the first measuring electrode 28. The electromotive force, which is generated between the first measuring electrode 28 and the first reference electrode 30, is measured by the first potentiometer 32.

The control of the electrochemical pumping cell is corrected by regulating a target value of partial pressure of oxygen in the atmosphere in the first internal space 6 concerning the second electrochemical sensor cell which controls the electrochemical pumping cell so that the electromotive force of the third electrochemical sensor cell is maintained to be a constant value. For example, when the partial pressure of oxygen in the atmosphere in the second internal space 8 is raised, namely when the electromotive force of the third electrochemical sensor cell is lowered, the target value of partial pressure of oxygen is lowered. Thus the electrochemical pumping cell is operated so that the partial pressure of oxygen in the atmosphere in the first internal space 6 is lowered. Accordingly, the partial pressure of oxygen in the atmosphere in the first internal space 6, and consequently the partial pressure of oxygen in the atmosphere in the second internal space 8 are lowered.

More specifically, a system as shown in FIG. 4 (B) is employed. At first, a voltage value of the third potentiometer 64 is compared with a comparison voltage corresponding to a target value of partial pressure of oxygen in the atmosphere in the second internal space 8, by using a comparator. A difference therebetween is amplified into a predetermined magnitude by using an amplifier, followed by being subjected to a delaying and waveform-converting process to avoid oscillation upon feedback control so that a correcting comparison voltage is obtained. The correcting comparison voltage is applied in series to a comparison voltage for the second potentiometer 26 which corresponds to a target value of partial pressure of oxygen in the atmosphere in the first internal space 6. Thus the comparison voltage for the second potentiometer 26 is corrected. After that, a voltage value of the second potentiometer 26 is compared with the corrected comparison voltage for the second potentiometer 26 by using a comparator. A difference therebetween is amplified by an amplifier into a predetermined magnitude to obtain a voltage which is applied to the electrochemical pumping cell so that the electrochemical pumping cell is operated.

According to the NOx sensor as described above, even when the oxygen concentration in the measurement gas is high, and hence the change in electromotive force of the first electrochemical sensor cell, i.e., the measuring error for the NOx concentration, which is caused by the change in distribution of oxygen concentration in the measurement gas depending on the oxygen concentration in the measurement gas, is extremely large, the electrochemical pumping cell is controlled by the second electrochemical sensor cell on the basis of the value of partial pressure of oxygen in the atmosphere in the vicinity of the first electrochemical sensor cell. Accordingly, the value of partial pressure of oxygen is stably maintained to be constant. Therefore, it is possible to further decrease the measuring error for the NOx concentration resulting from the distribution of oxygen concentration in the measurement gas. Moreover, the porous material layer 62 having the predetermined diffusion resistance is provided around the first measuring electrode 28 of the first electrochemical sensor cell. Accordingly, the sensitivity for detecting the NOx concentration is also improved.

In the foregoing first sensor, the concentration of NOx is determined by detecting the change in partial pressure of oxygen in the second internal space caused by oxygen produced by reducing or decomposing NOx in the second internal space, as the change in value of the electromotive force outputted by the first electrochemical sensor cell. However, a NOx sensor and a method of measuring NOx as described below are also advantageously adopted in order to measure the NOx concentration in the measurement gas.

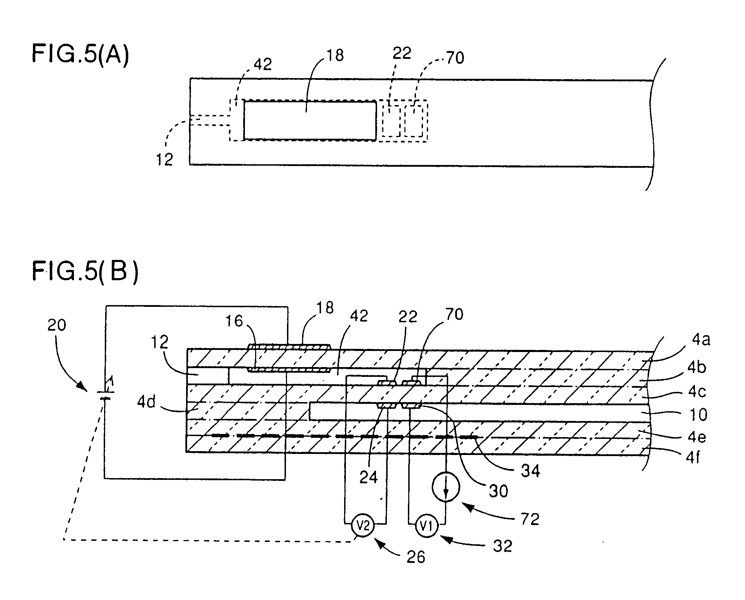

Namely, an electrochemical sensor cell is formed, which includes a first measuring electrode arranged in a predetermined internal space, having a porous structure and functioning as a NOx-reducing catalyst as well. A constant current is allowed to flow so that oxygen is pumped out from the first measuring electrode to a first reference electrode, by using a constant current power source provided on an electromotive force-outputting circuit comprising the first measuring electrode and the first reference electrode of the electrochemical sensor cell. Under this condition, NOx existing in an atmosphere in the internal space is reduced or decomposed in a porous structure of the first measuring electrode. An electromotive force, which corresponds to a partial pressure of oxygen in an atmosphere in the porous structure, defined by oxygen produced by the reduction or decomposition of NOx, is detected from an output from the electrochemical sensor cell to obtain a detected value from which a NOx concentration in a measurement gas is determined. An example is shown in FIG. 5 (A), (B).

Another NOx sensor (second NOx sensor) is shown in FIG. 5 (A), (B). The second sensor adopts a structure similar to that shown in FIG. 2 (A) and FIG. 2 (B), which is specifically **characterized in that** the function of the porous material layer 40 in FIG. 2 (A), (B) is possessed by a first measuring electrode 70, and a constant current power source is provided on an electromotive force-outputting circuit comprising the first measuring electrode and the first reference electrode of the electrochemical sensor cell in FIG. 2 (A), (B) to allow a constant current to flow so that oxygen is pumped out from the first measuring electrode to the first reference electrode, and thus a partial pressure of oxygen in an atmosphere in a porous structure of the first measuring electrode comes to a predetermined value at which NOx is reduced.

Namely, in the second sensor, the first measuring electrode 70 having the porous structure also functions as both of the second diffusion rate-determining means and the second internal space. At first, the atmosphere of the measurement gas with its partial pressure of oxygen controlled in an internal space 42 is introduced into the inside of the first measuring electrode 70 having the porous structure under a predetermined diffusion resistance.

After that, the oxygen in the atmosphere of the measurement gas introduced into the inside of the first measuring electrode 70 having the porous structure is pumped out from the inside of the first measuring electrode 70 to a reference gas-existing space 10 by the aid of the first reference electrode 30, in a flow amount always corresponding to a previously set current value of the constant current power source, owing to the pumping action effected by the constant current power source 72 provided on the electromotive force-outputting circuit comprising the first measuring electrode 70 and the first reference electrode 30 of the first electrochemical sensor cell. However, the first measuring electrode 70 has the porous structure having the diffusion resistance. Accordingly, pressure loss occurs in the flow of oxygen caused by diffusion effected by the pumping action of the constant current power source 72. The partial pressure of oxygen in the porous structure is lower than that in the internal space 42 by an amount corresponding to the pressure loss.

NOx in the atmosphere diffuses into the first measuring electrode 70 under the predetermined diffusion resistance. NOx is reduced or decomposed, in the vicinity of the surface of the first measuring electrode 70, by the first measuring electrode 70 having the porous structure which also functions as a NOx-reducing catalyst. The partial pressure of oxygen in the atmosphere in the porous structure of the first measuring electrode 70 is increased by oxygen produced by the reduction or decomposition of NOx. When the NOx concentration is zero, the partial pressure of oxygen in the porous structure is lower than that in the internal space 42 by the amount corresponding to the pressure loss. Since the first measuring electrode 70 has the diffusion resistance, the produced oxygen in the first measuring electrode scarcely diffuses into the internal space 42 when the amount of increase in partial pressure of oxygen is smaller than the amount corresponding to the pressure loss as a matter of course, and even when the former is larger than the latter. Therefore, the change in NOx concentration in the measurement gas results in a large change in partial pressure of oxygen in the porous structure.

The constant current power source 72 greatly changes the voltage to be applied to the first electrochemical sensor cell, in order to allow the current of the previously set value to continuously flow through the electromotive force-outputting circuit in response to the change in partial pressure of oxygen in the porous structure of the first measuring electrode 70. The change in voltage, which is applied to the first electrochemical sensor cell by the constant current power source 72 corresponding to the partial pressure of oxygen in the atmosphere in the porous structure, defined by the produced oxygen, is detected by a first potentiometer 32 provided on the electromotive force-outputting circuit of the first electrochemical sensor cell. Accordingly, even a slight amount of produced oxygen can be measured as a large change in voltage. Moreover, the diffusion of produced oxygen into the internal space is suppressed to be less than that of the NOx sensor concerning the first embodiment. Thus the sensitivity for detecting the NOx concentration is more increased than that of the NOx sensor previously described.

Desirably, the partial pressure of oxygen in the internal space 42 is set to be not more than about 1/100 of the NOx concentration in the measurement gas in order to increase the sensitivity for detecting the NOx concentration. If NOx is reduced in the internal space 42 in this procedure, an alloy cermet electrode composed of, for example, Pt/Au is used as the second measuring electrode 22 and the inner pumping electrode 16 in order to lower the ability to reduce NOx.

In the NOx sensor having the structure as described above, the first measuring electrode 70 having the porous structure also functions as the second diffusion rate-determining means and the second internal space. Therefore, it is unnecessary to separately provide a diffusion rate-determining means. Thus the structure of the NOx sensor is simplified, providing an advantage from the viewpoint of cost and applicability to mass production. Moreover, when a diffusion rate-determining means, which is, for example, a diffusion rate-determining layer composed of a porous material layer, is separately provided on the first measuring electrode 70, it is possible to more effectively suppress the diffusion of oxygen produced by the reduction or decomposition of NOx, from the inside of the first measuring electrode 70 to the internal space 42. Thus the sensitivity for detecting the NOx concentration is further improved.

As for the second NOx sensor, in order to decrease the change in electromotive force of the first electrochemical sensor cell, i.e., the measuring error for the NOx concentration, caused by the change in distribution of oxygen concentration existing in the gas-diffusing direction depending on the oxygen concentration in the measurement gas, it is desirable that the first measuring electrode 70 and the second measuring electrode 22 are arranged in parallel with respect to the direction of diffusion of the atmosphere in the internal space 42, in the same manner as the second sensor.

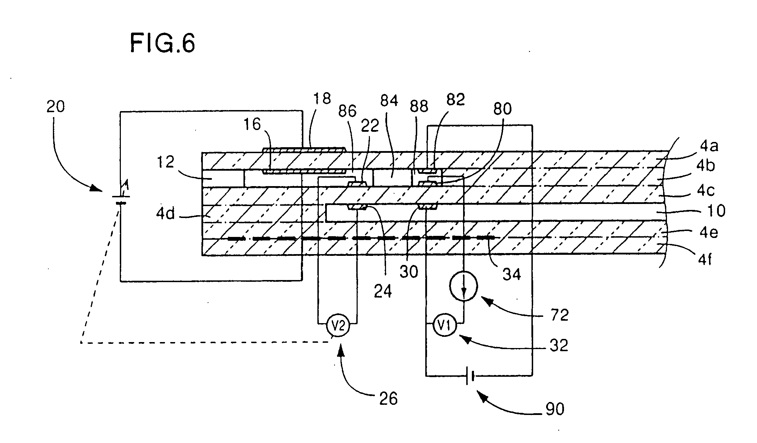

A modified form of the second sensor, shown in FIG. 6 is different from the form shown in FIG. 5 (A) and FIG. 5 (B), which is characterized as follows. Namely, the internal space 42 in FIG. 5 (A) and FIG. 5 (B) is divided into a first internal space 86 communicating with the external measurement gas-existing space through a first diffusion rate-determining passage 12, and a second internal space 88 into which an atmosphere in the first internal space 86 is introduced through a second diffusion rate-determining passage 84 under a predetermined diffusion resistance. The electrochemical pumping cell is located on the first internal space 86, while the first electrochemical sensor cell is located on the second internal space 88. An inner subsidiary pumping electrode 82 is provided in contact with the solid electrolyte layer 4a located on the second internal space 88. The inner subsidiary pumping electrode 82, the solid electrolyte layers 4a, 4b, 4c, and the first reference electrode 30 constitute a subsidiary oxygen-pumping cell for lowering a partial pressure of oxygen in the atmosphere introduced from the first internal space 86 into the second internal space 88 to a degree sufficient to reduce NOx in a porous structure of a first measuring electrode 80 of the first electrochemical sensor cell. The subsidiary oxygen-pumping cell is operated by an external DC power source 90.

Namely, the measurement gas introduced from the first internal space 86 through the second diffusion rate-determining passage 84 undergoes the pumping action effected by the subsidiary oxygen-pumping cell constructed by the inner subsidiary pumping electrode 82 arranged in the second internal space 88, the first reference electrode 30, and the solid electrolyte layers 4a, 4b, 4c. Thus the second internal space 88 is controlled to have a low and constant value of partial pressure of oxygen. The low and constant value of partial pressure of oxygen is stably maintained to be constant even when the oxygen concentration in the measurement gas is high, and hence the change in electromotive force of the first electrochemical sensor cell, i.e., the measuring error for the NOx concentration, caused by the change in distribution of oxygen concentration in the measurement gas depending on the oxygen concentration in the measurement gas, is extremely large. Accordingly, it is possible to further decrease the measuring error for the NOx concentration, caused by the distribution of oxygen concentration in the measurement gas. Moreover, the NOx concentration in the measurement gas can be accurately measured in a state in which the partial pressure of oxygen in the first internal space 86 is previously set to be a predetermined value which is sufficiently higher than that in the second internal space 88. Accordingly, even when inflammable gases such as CO, HC, and H₂ are present in the measurement gas in a mixed manner, the inflammable gases can be removed by oxidation in the first internal space 86. Thus it is possible to decrease the measuring error for the NOx concentration, due to interference by the inflammable gases in the second internal space 88, as small as possible. It is needless to say that the subsidiary oxygen-pumping cell can be equivalently applied and installed for the NOx sensor (first NOx sensor) according to the present invention, and thus an equivalent effect can be obtained.

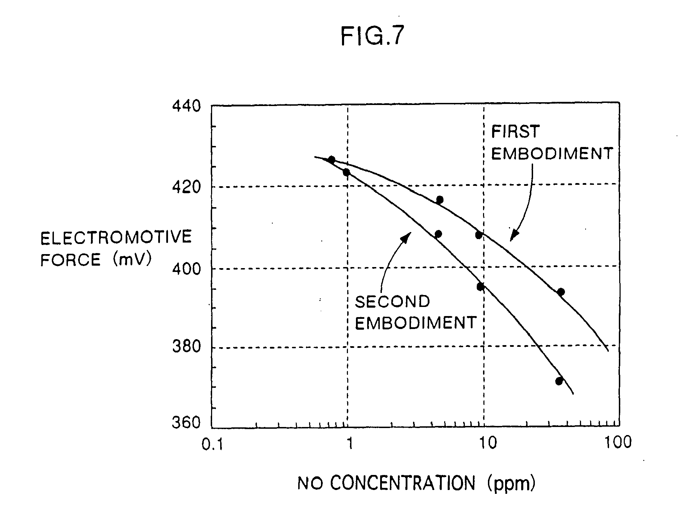

The NOx sensors of the first and second embodiments shown in FIG. 1 (A), (B) and FIG. 5 (A) and FIG. 5 (B) respectively were used under the following condition. Namely, the pumping voltage of the electrochemical pumping cell was 430 mV. The partial pressure of oxygen in the atmospheres in the first internal space 6 or the internal space 42 was controlled to be 10⁻¹⁰ atm. In this state, NO as a NOx component in a measurement gas comprising 5 % O₂ in a carrier gas of N₂ was changed in a range of 0.8 to 40 ppm. The change in electromotive force between the first measuring electrode 28 or 70 and the reference electrode 30, obtained under this condition, is shown in FIG. 7. As clarified from the result shown in FIG. 7, the electromotive force changes in a range of about 15 mV in the first embodiment, or in a range of about 30 mV in the second embodiment, in a range of NO concentration of 1 to 10 ppm. Accordingly, a large change in signal can be detected by the first potentiometer 32 even when the NO concentration in the measurement gas is low.

When the NO concentration is determined by measuring the electromotive force as performed by the NOx sensor according to the present invention, it is possible to select the sensitivity to NO and the measuring range by controlling the diffusion resistance concerning the first measuring electrode 28. As for the result shown in FIG. 7, the first embodiment concerns a case of the use of a sum of diffusion resistances of the diffusion rate-determining passage 14 having a cross-sectional area of 0.2 mm² and the measuring electrode 28 itself comprising a porous cermet having an electrode thickness of 10 µm and a porosity of 40 %. The second embodiment concerns a case of the use of only a diffusion resistance of the measuring electrode 70 itself comprising a porous cermet having an electrode thickness of 10 µm and a porosity of 40 %. The sensitivity to NO is increased by increasing the diffusion resistance by providing, for example, a porous diffusion rate-determining section such as a porous alumina layer and a porous zirconia layer stacked on the measuring electrode 28, 70. However, it is difficult to perform measurement in a high concentration region. O₂ produced by reduction on the electrode is apt to remain in the electrode area, and the oxygen concentration is apt to increase. Therefore, the change in electromotive force becomes large. However, the oxygen concentration in the electrode area is apt to increase due to O₂ produced by reduction. For this reason, reduction does not occur if the oxygen concentration exceeds a certain level. Accordingly, it is difficult to perform measurement in a high concentration region. Therefore, the sensitivity and the measuring range should be determined by appropriately setting the diffusion resistance depending on the feature of a region in which the NO concentration is measured.

As clarified from the foregoing explanation, according to the method of measuring, even when the NOx concentration in a measurement gas is low in a degree of several ppm, an electromotive force is detected, which corresponds to a partial pressure of oxygen in an atmosphere, defined by oxygen produced by reducing or decomposing NOx, in the presence of the diffusion rate-determining means having a predetermined diffusion resistance. Thus a large change in electromotive force, i.e., a large change in signal is obtained as exemplified in FIG. 7 even when the measurement gas component has a low concentration. Moreover, measurement can be performed continuously and accurately with good response over a long period of time.

## Claims

1. A method of measuring a concentration of NO as a gas component of a measurement gas, comprising the steps of:
introducing the measurement gas containing the NO component from an external measurement-gas space into a first internal space (6) under a first diffusion resistance;
controlling the partial pressure of oxygen in said first internal space (6) to a value at which NO is not reduced;
introducing the atmosphere from said first internal space (6) into a second internal space (8) under a second diffusion resistance;
reducing or decomposing NO in said second internal space (8) to produce oxygen;
detecting the amount of oxygen produced by reducing or decomposing NO solely within said second internal space on the basis of an electromotive force generated due to oxygen concentration at an electrochemical sensor cell (4c, 28, 30) arranged in said second internal space; and
determining the concentration of NO in the measurement gas by measuring said amount of oxygen.

## Patentansprüche

1. Verfahren zur Messung einer Konzentration von NO als Gaskomponente eines Messgases, das folgende Schritte umfasst:
das Einbringen des Messgases, das die NO-Komponente enthält, aus einem externen Messgasraum in einen ersten Innenraum (6) unter einem ersten Diffusionswiderstand;
das Steuern des Sauerstoffpartialdrucks in dem ersten Innenraum (6), sodass dieser einen Wert aufweist, bei dem NO nicht reduziert wird;
das Einbringen der Atmosphäre aus dem ersten Innenraum (6) in einen zweiten Innenraum (8) unter einem zweiten Diffusionswiderstand;
das Reduzieren oder Zersetzen von NO in dem zweiten Innenraum (8) zur Erzeugung von Sauerstoff;
das Detektieren der durch das Reduzieren oder Zersetzen von NO erzeugten Sauerstoffmenge ausschließlich in dem zweiten Innenraum basierend auf einer elektromotorischen Kraft, die aufgrund der Sauerstoffkonzentration erzeugt wird, durch eine in dem zweiten Innenraum angeordnete, elektrochemische Abtastzelle (4c, 28, 30) und
das Bestimmen der NO-Konzentration in dem Messgas durch die Messung der Sauerstoffmenge.

## Revendications

1. Procédé de mesure d'une concentration de NO en tant que composant gazeux dans un gaz à mesurer, comprenant les étapes de:
introduire le gaz à mesurer contenant le composant NO depuis un espace de gaz de mesure externe dans un premier espace interne (6) sous une première résistance à la diffusion;
commander la pression partielle de l'oxygène dans ledit premier espace interne (6) à une valeur à laquelle NO n'est pas réduit;
introduire l'atmosphère dudit premier espace interne (6) dans un deuxième espace interne (8) sous une deuxième résistance à la diffusion;
réduire ou décomposer NO dans ledit deuxième espace interne (8) pour produire de l'oxygène;
détecter la quantité d'oxygène produite en réduisant ou en décomposant NO uniquement dans ledit deuxième espace interne sur la base d'une force électromotrice produite en raison de la concentration de l'oxygène à une cellule de capteur électrochimique (4c, 28, 30) agencée dans ledit deuxième espace interne; et
déterminer la concentration de NO dans le gaz de mesure en mesurant ladite quantité d'oxygène.
